(19) Europäisches Patentamt
European Patent Office
Office européen des brevets

(11) **EP 3 764 370 B1**

(12) **FASCICULE DE BREVET EUROPEEN**

(45) Date de publication et mention de la délivrance du brevet:
**04.09.2024 Bulletin 2024/36**

(21) Numéro de dépôt: **19186032.9**

(22) Date de dépôt: **12.07.2019**

(51) Classification Internationale des Brevets (IPC):
**G16H 50/80** (2018.01)   **G16B 20/20** (2019.01)

(52) Classification Coopérative des Brevets (CPC):
**G16H 50/80**

(54) **PROCÉDÉ D'IDENTIFICATION ET DE SURVEILLANCE ÉPIDÉMIOLOGIQUE D'UN FOYER BACTÉRIEN**

EPIDEMIOLOGISCHES IDENTIFIZIERUNGS- UND ÜBERWACHUNGSVERFAHREN EINES BAKTERIENHERDES

METHOD FOR IDENTIFICATION AND EPIDEMIOLOGICAL SURVEILLANCE OF A BACTERIAL FOCUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**13.01.2021 Bulletin 2021/02**

(73) Titulaire: **bioMérieux**
**69280 Marcy l'Etoile (FR)**

(72) Inventeurs:
• **KANEKO, Gaël**
**69280 Marcy l' Etoile (FR)**
• **GUIGON, Ghislaine**
**69570 Dardilly (FR)**

(74) Mandataire: **bioMérieux PI Groupement mandataires**
**bioMérieux**
**69280 Marcy l'Etoile (FR)**

(56) Documents cités:
• **MARJOLEIN F. Q. KLUYTMANS-VAN DEN BERGH ET AL: "Whole-Genome Multilocus Sequence Typing of Extended-Spectrum-Beta-Lactamase-Producing Enterobacteriaceae", JOURNAL OF CLINICAL MICROBIOLOGY, vol. 54, no. 12, 14 September 2016 (2016-09-14), US, pages 2919 - 2927, XP055656833, ISSN: 0095-1137, DOI: 10.1128/JCM.01648-16**

• **JAMES STIMSON ET AL: "Abstract", BIORXIV, 3 December 2018 (2018-12-03), XP055656344, Retrieved from the Internet <URL:https://www.biorxiv.org/content/10.1101/319707v3.full.pdf> [retrieved on 20200109], DOI: 10.1101/319707**

• **MICHAEL INOUYE ET AL: "Short read sequence typing (SRST): multi-locus sequence types from short reads", BMC GENOMICS, BIOMED CENTRAL, vol. 13, no. 1, 24 July 2012 (2012-07-24), pages 338, XP021115188, ISSN: 1471-2164, DOI: 10.1186/1471-2164-13-338**

• **SURESH BABU ET AL: "Various performance measures in Binary classification -An Overview of ROC study", INTERNATIONAL JOURNAL OF INNOVATIVE SCIENCE, ENGINEERING & TECHNOLOGY, 1 September 2015 (2015-09-01), XP055657111, Retrieved from the Internet <URL:http://ijiset.com/vol2/v2s9/IJISET_V2_I9_72.pdf> [retrieved on 20200113]**

- WALKER TIMOTHY M ET AL: "Whole-genome sequencing to delineate Mycobacterium tuberculosis outbreaks: a retrospective observational study", THE LANCET INFECTIOUS DISEASES, vol. 13, no. 2, 15 November 2012 (2012-11-15), AMSTERDAM, NL, pages 137 - 146, XP093129695, ISSN: 1473-3099, Retrieved from the Internet <URL:https://pdf.sciencedirectassets.com/272254/1-s2.0-S1473309913X70525/1-s2.0-S147330991 2702773/main.pdf?X-Amz-Security-Token=IQo Jb3JpZ2luX2VjED0aCXVzLWVhc3QtMSJGMEQ CIBV4HRbLol7MRHvKUMwDDtZhl7bLiMTt6rE/t Ulv/GJGAiAmwE+LHKdwhVrriz68ASLUkFiqYvG YQ5oFhj6AQ8vKDiqzBQgWEAUaDDA1OTAwMz U0Njg2NSIMqWxssgo4OsSCEblnK> DOI: 10.1016/S1473-3099(12)70277-3

**Description**

**DOMAINE DE L'INVENTION**

La présente invention a trait au domaine de l'épidémiologie bactérienne, en particulier la détection et la surveillance de foyer bactériens en fonction du génomes de souches bactériennes, notamment le séquençage partiel ou total de l'ADN et/ou de l'ARN des souches bactériennes.

**ETAT DE LA TECHNIQUE**

La détection d'un foyer infectieux (ou « outbreak ») bactérien consiste classiquement à déterminer si plusieurs souches bactériennes prélevées chez des sujets (e.g. des patients et par extension chez des animaux) résultent d'une transmission récente d'une même souche entre les sujets, par exemple la transmission de la souche à plusieurs sujets depuis un sujet « source » ou la transmission de la souche de sujet à sujet. En se fondant sur les outils microbiologiques classiques, la détection est usuellement réalisée en deux temps :

a. dans un premier temps en suspectant un foyer bactérien, cette suspicion survenant lorsque des souches prélevées appartiennent à la même espèce bactérienne et partagent des caractéristiques phénotypiques communes, par exemple un antibiogramme identique ou voisin pour les bactéries pathogènes ;

b. et en cas de suspicion en menant une enquête épidémiologique visant à démontrer, ou infirmer, que ces souches résultent bien d'une transmission entre sujets. Ce type d'enquête consiste notamment à rechercher si les sujets objet des prélèvements ont été récemment en contact, ont partagés une même localisation (e.g. une même salle d'opération ou une même chambre dans un hôpital), ont été soignés par un même personnel soignant, etc. Ce type d'enquête est le plus souvent long et fastidieux, mobilise beaucoup de personnes. En outre, une enquête peut perturber grandement le fonctionnement d'une institution ou d'une société suspectée d'être l'objet d'une épidémie dans la mesure où des mesures prophylactiques sont le plus souvent mise en oeuvre avant la fin de l'enquête, comme par exemple la mise en quarantaine d'une chambre, d'un service ou la fermeture d'une salle d'opération.

Dans ce contexte, l'avènement du séquençage, en particulier les séquençages du type WGS (« whole genome sequencing », séquençage de génome entier en français) représente une avancée notable dans l'épidémiologie bactérienne puisqu'un génome bactérien entier contient un niveau d'information bien plus important que celui délivré par les techniques microbiologiques classiques. Non seulement, les critères pour décider de lancer une étude épidémiologiques sont plus précis mais de plus l'emploi de la génomique peut également grandement simplifier et normaliser celle-ci. Par exemple, si deux souches de staphylocoques dorés, prélevées au sein du même service hospitalier à quelques jours d'intervalle, sont strictement identiques du point de vue génomique, il peut être déterminé sans autre information que les deux souches font bien partie d'un même foyer bactérien.

Si le séquençage se révèle une avancée notable, il ne permet cependant pas à lui seul de déterminer la lignées de deux souches bactériennes quel que soit l'espèce. En effet, certaines espèces bactériennes ont un génome plastique évoluant très rapidement en l'espace de quelques jours, et ce d'autant plus qu'un traitement antibiotique est mis en oeuvre, de sorte qu'une stricte identité entre génomes ne peut être utilisée comme seul critère. Pour tenir compte de cette plasticité, des procédés de détection de foyers bactériens consistent à évaluer que des souches bactériennes appartiennent à un même foyer si leur différence génomique, par exemple calculée en fonction du nombre de polymorphismes d'un seul nucléotide (ou « single-nucleotide polymorphisme en anglais), est inférieure à un seuil prédéterminé. Comme décrit dans l'article « Beyond the SNP threshold : identifying outbreak clusters using inferred transmission » de J. Simson et al, dec. 2018, cette approche est cependant peu précise en raison de nombreuses sources d'incertitude, comme par exemple le contexte dans lequel évoluent les bactéries ou la variabilité du taux de mutation en fonction des espèces. Les auteurs de cet article proposent ainsi de tenir compte également de la chronologie des prélèvements d'échantillon contenant les souches bactériennes et de connaissances *a priori* sur les mécanismes de mutation et de transmission des souches bactériennes.

Outre la complexification des modèles de prédiction épidémiologiques, l'emploi d'un unique seuil mène nécessairement à un compromis difficile entre sensibilité et spécificité de la prédiction. D'un côté si la prédiction d'appartenance à un foyer bactérien est trop sensible mais trop peu spécifique, le déclenchement des enquêtes épidémiologiques menant à infirmer le caractère épidémique d'un évènement est trop fréquent, ce qui implique un cout important en termes de ressources, de fonctionnement et de budget. D'un autre côté si la prédiction d'appartenance à un foyer est peu sensible, alors des foyers bactériens ne sont pas détectés, avec des conséquences graves en termes de santé, par exemple celle de patients ou de consommateurs L'article de Walker Timothy M et al: "Whole-genome sequencing to delineate Mycobacterium tuberculosis outbreaks: a rétrospective observational study",The Lancet Infectious Diseases, vol. 13, no. 2, 15 novembre 2012 (2012-11-15), pages 137-146, concerne la classification d'une souche bactérienne comme appartenant, non-appartenant et appartenant peut-être à un foyer bactérien dans le contexte d'une épidémie d'infection bactérienne.

## EXPOSE DE L'INVENTION

La présente invention est définie par les revendications.

Le but de la présente invention est de proposer un procédé d'identification et de surveillance d'un foyer bactérien à base de comparaison de génomes bactériens qui offre une liberté en termes de sensibilité et de spécificité tout en tenant compte explicitement des sources d'incertitude dans la prédiction d'appartenance de souches bactériennes au foyer bactérien.

A cet effet, l'invention a pour objet un procédé de détection et de surveillance d'un foyer bactérien lié à une espèce bactérienne au sein d'une zone géographique, comprenant :

- l'obtention d'un génome numérique d'une souche bactérienne prélevée au sein de la zone géographique et appartenant à l'espèce bactérienne ;
- le calcul d'une distance génomique du génome numérique obtenu avec un génome numérique d'une base de données, dite « épidémiologique », comprenant au moins un génome numérique d'une souche bactérienne appartenant à l'espèce bactérienne;
- la prédiction :

    ∘ que la souche bactérienne prélevée et la souche bactérienne de la base de données appartiennent au foyer bactérien si leur distance génomique est inférieure à un premier seuil prédéterminé ; ou
    ∘ que la souche bactérienne prélevée et la souche bactérienne de la base de données n'appartiennent pas au foyer bactérien si leur distance génomique est supérieure à un second seuil prédéterminé strictement supérieur au premier seuil ; ou
    ∘ que la souche bactérienne prélevée et la souche bactérienne de la base de données appartiennent peut être au foyer bactérien si leur distance génomique est comprise entre le premier et le second seuil ;

procédé selon lequel :

- le premier seuil est supérieur ou égal à un troisième seuil tel qu'une prédiction d'appartenance au foyer bactérien de deux souches bactériennes ayant une distance génomique inférieure au troisième seuil a une spécificité maximale ; et
- le second seuil est inférieur ou égal à un quatrième seuil tel qu'une prédiction de non appartenance au foyer bactérien de deux souches bactériennes ayant une distance génomique supérieure au quatrième seuil a une sensibilité maximale.

En d'autres termes, deux seuils différents sont utilisés pour régler la sensibilité et la spécificité du procédé, le seuil le plus bas étant utilisé pour régler la spécificité de la prédiction d'appartenance d'une souche au foyer bactérien (ci-après « spécificité d'appartenance ») et le seuil le plus haut étant utilisé pour régler la sensibilité de cette prédiction (ci-après « sensibilité d'appartenance »). La zone comprise entre ces deux seuils est ainsi spécifiquement prévue pour tenir compte des incertitudes inhérentes à une prédiction basée sur des distances génomiques. En particulier les troisième et quatrième seuils, préalablement appris pour maximiser la spécificité et la sensibilité d'appartenance, définissent une zone où il est difficile de savoir si des souches appartiennent ou non à un même foyer en raison de données incomplètes ou trop peu diversifiées pour apprendre ces seuils, de méconnaissance des mécanismes de mutation qui sont hétérogènes au sein de l'espèce bactérienne, d'une imprécision du procédé en raison du choix de la méthode de comparaison génomique ou encore des erreurs de caractérisation des foyers infectieux résultant des enquêtes épidémiologiques. Cette zone d'incertitude offre à l'utilisateur une souplesse dans la gestion des épidémies. En particulier, contrairement à la prédiction d'appartenance au foyer bactérien qui déclenche une enquête épidémiologique et des mesures prophylactiques pour endiguer le foyer bactérien, lorsqu'une souche est dans la zone intermédiaire, l'utilisateur peut mettre en place une enquête préliminaire, par exemple en recoupant avec le dossier du patient sur lequel le prélèvement a été réalisé ou en analysant son résistome, son virulome ou sa position phylogénique dans la biodiversité de l'espèce, pour décider si oui ou non une enquête épidémiologique poussée doit être mise en oeuvre. Par ailleurs, la zone comprise entre les troisième et quatrième seuils peut dans certain cas être trop importante de sorte que la prédiction basée sur ces seuils n'est pas optimale. Les premier et second seuils, définissant une zone strictement comprise entre les troisième et quatrième seuils, autorisent une optimisation analytique de la prédiction d'appartenance ou de non appartenance au foyer bactérien.

Selon un mode de réalisation, le premier et le second seuils sont égaux à deux distances génomiques calculées :

- en constituant une base de données d'apprentissage de génomes numériques de souches bactériennes appartenant à l'espèce bactérienne, ladite base comprenant :

  ○ des couples de souches bactériennes préalablement déterminées comme appartenant à un même foyer bactérien, et étiquetés comme « couples de souches reliées » ;
  ○ des couples de souches bactériennes préalablement déterminée comme n'appartenant pas à un même foyer bactérien, et étiquetés comme « couples de souches non reliées » ;

- en choisissant un prédicteur binaire configuré pour prédire que deux souches bactériennes sont reliées ou non reliées par comparaison de leur distance génomique à un cinquième seuil ;
- pour chaque valeur de cinquième seuil appartenant à un ensemble prédéterminé de valeurs de cinquième seuil, en calculant

  ○ une matrice de confusion dudit prédicteur en fonction de la base de données d'apprentissage ;
  ○ un premier index de qualité du prédicteur en fonction de la matrice de confusion, ledit premier index étant différent de la sensibilité et de la spécificité du prédicteur ;
  ○ un second index de qualité, différent du premier index, en fonction de la matrice de confusion, ledit second index étant différent du premier index, de la sensibilité et de la spécificité du prédicteur ;

- en recherchant une première valeur de cinquième seuil qui optimise le premier index et une seconde valeur de cinquième seuil qui optimise le second index ;
- en posant le premier seuil comme égal au minimum de la première et de la seconde valeurs de cinquième seuil et en posant le second seuil comme égal au maximum de la première et de la seconde valeurs de cinquième seuil.

En d'autres termes, une prédiction basée sur une spécificité et une spécificité d'appartenance maximales ne constituent pas nécessairement une prédiction optimale au regard des données épidémiologiques disponibles, stockées dans la base de données d'apprentissage. En calculant des premier et second seuils qui optimisent la qualité de la prédiction binaire, il est obtenu de fait une optimisation de la gestion des évènements épidémiques, tout en conservant une zone intermédiaire suffisamment large pour continuer d'alerter l'utilisateur d'un possible foyer bactérien.

Selon un mode de réalisation, le premier index est choisi pour tenir compte du déséquilibre, dans la base de données d'apprentissage, entre le nombre de couples de souches reliées et le nombre de couples de souches reliées. En particulier, le premier index est le coefficient de corrélation de Matthews ou le F1 score. D'une manière générale, les données concernant les foyers bactériens, c'est-à-dire le nombre de souches considérées comme reliées, sont bien moins nombreuses que les souches considérées comme non reliées. En utilisant un index de qualité qui prend explicitement en compte ce déséquilibre, une meilleure optimisation de la prédiction est obtenue. En outre, le seuil correspondant au coefficient de Matthews ou du F1-score privilégie la spécificité sans pour autant ne prendre que la spécificité en compte.

Selon un mode de réalisation, le second index est l'index de Youden. Cet index, qui prend explicitement en compte la spécificité et la sensibilité permet naturellement d'optimiser la prédiction de non appartenance dont l'apprentissage est réalisée usuellement sur une donnée importante. Le déséquilibre de la base de données a pour effet que l'index de Youden est plus influencé par la sensibilité, la spécificité étant proche de 1 sur tout l'intervalle compris entre les troisième et quatrième seuils.

Selon un mode de réalisation, le prédicteur est choisi de sorte que :

- les vrais positifs correspondent aux couples de souches reliées ayant une distance génomique inférieure au cinquième seuil :
- les faux négatifs correspondent aux couples de souches reliées ayant une distance génomique supérieure au cinquième seuil ;
- la faux positifs correspondent aux couples de souches non reliées ayant une distance génomique inférieure au cinquième seuil ; et
- les vrais négatifs correspondent aux couples de souches non reliées ayant une distance génomique supérieure au cinquième seuil.

Selon un mode de réalisation, la base de données épidémiologique comprend la base de données d'apprentissage. En d'autres termes, la base de données d'apprentissage est complété à mesure que le procédé est mise en oeuvre, ce qui permet de raffiner les différents seuils à mesure que la base augmente.

Selon un mode de réalisation, la distance génomique est une distance normalisée. Plus particulièrement, la distance génomique entre deux souches bactériennes est calculée en :

- sélectionnant, dans un ensemble prédominé de loci, les loci communs aux génomes numériques desdites souches ;
- comptant le nombre de différences alléliques, aux loci communs, entre les deux génomes numériques desdites souches ;
- en divisant ledit nombre de différences par le nombre de loci communs.

En normalisant par le nombre de loci en communs, l'impact des erreurs de séquençage, en particulier le fait de ne pas identifier un loccus chez une souche bactérienne, est atténué.

Selon un mode de réalisation privilégié, si les premières et secondes valeurs de cinquième seuil sont supérieures à 0,1, alors :

- le second seuil est posé égal à 0,1 ;
- le premier seuil est posé égal à max($D_g \backslash D_g < 0,2$), où max($D_g \backslash D_g < 0,2$) est la plus grande distance génomique, parmi les couples de souches reliées, strictement inférieure à 0,2.

En particulier, les inventeurs ont constaté que des valeurs supérieures à 0,1, obtenues usuellement en raison d'une base de données d'apprentissage incomplète ou trop peu diverse, matérialise un échec de l'apprentissage. Les inventeurs ont de plus noté que dans le cadre d'une base de données d'apprentissage appropriée, les premier et second seuils sont inférieurs ou égaux à 0,1. Un des deux seuils est ainsi fixé à cette borne supérieure. Par ailleurs, les inventeurs ont constaté que deux souches de même sous-types ont en très grande majorité une distance génomique inférieure à 0,2. Ainsi en posant l'autre seuil égal à max($d_r \backslash d_r < 0,2$), deux souches de distance génomique supérieure à ce dernier, il est prédit que ces souches n'appartiennent pas au même sous-types bactérien, et donc n'appartiennent pas au même foyer, ce qui constitue un indice important pour la suspicion d'épidémie. Ainsi, alors même que les données sont encore insuffisantes pour calculer avec précision les premier et second seuils, l'utilisateur dispose d'un procédé par défaut.

Selon un mode de réalisation, les distances entre les génomes numériques sont calculées en fonction d'un base de marqueurs, en particulier une base wgMLST, cgMLST, MLST, de gènes ou de SNP.

Selon un mode de réalisation, lorsqu'une souche prélevée est prédite comme appartenant au foyer bactérien, elle est étiquetée dans la base de donnée épidémiologique comme étant « reliée » avec les souches bactériennes du foyer bactérien et comme étant « non reliée » avec les autres souches bactériennes.

Selon un mode de réalisation, lorsqu'une souche prélevée est prédite comme appartenant peut-être au foyer bactérien, une caractérisation supplémentaire de ladite souche est mise en oeuvre pour déterminer si elle appartient effectivement audit foyer, et si tel est le cas la souche bactérienne prélevée est étiquetée, dans la base de donnée épidémiologique, comme étant « reliée » avec les souches bactériennes du foyer bactérien et comme étant « non reliée » avec les autres souches bactériennes.

Selon un mode de réalisation, le premier et le second seuil sont recalculés régulièrement et/ou dès que N nouvelles souches sont ajoutées à la base de données épidémiologique, où *N* est un entier supérieur ou égal à 1.

Selon un mode de réalisation, lorsqu'une souche est prédite comme appartenant au foyer bactérien, des mesures prophylactiques sont mises en oeuvre pour enrailler ledit foyer.

**BREVE DESCRIPTION DES FIGURES**

L'invention sera mieux comprise à la lecture de la description qui va suivre, donnée uniquement à titre d'exemple, et faite en relation avec les dessins annexés, dans lesquels des références identiques désignent des éléments identiques, et dans lesquels :

- la figure 1 est un organigramme d'un mode de réalisation du procédé selon l'invention ;
- la figure 2 illustre une table de correspondance entre souches bactériennes mémorisées dans une base de données d'apprentissage ;
- la figure 3 est une matrice de confusion d'un prédicteur binaire prédisant l'état reliées ou non reliées de deux souches bactériennes ;
- la figure 4 illustre une distribution du nombre de couples de souches reliées et une distribution du nombre de couples de souches non reliées en fonction de leur distance génomique ainsi qu'un seuil Ti utilisé pour calculer la matrice de confusion de la figure 3;
- la figure 5 est un tracé illustrant différents seuils sur les distances génomiques utilisés par le procédé selon l'invention ;
- la figure 6 illustre un système informatique et de séquençage pour la mise en oeuvre du procédé selon l'invention ;
- les figures 7A et 7B sont des distributions du nombre de couples de souches non reliées (distribution supérieure) et du nombre de couples de souches reliées (distribution inférieure) pour l'espèce bactérienne *Clostridium difficile*, la figure 7B étant un agrandissement entre 0 et 0,1 de la figure 7A ;
- les figures 8A et 8B illustrent, pour l'espèce *Clostridium difficile,* les distances génomiques pour différentes valeurs optimales d'indice de qualité, dont la sensibilité, la spécificité, la précision, la justesse (« accuracy » en anglais, i.e. $(TP + TN)/(N + P)$), le F1 score, l'indice de Youden, et le coefficient de corrélation de Matthews, la figure 8B étant un agrandissement entre 0 et 0,1 de la figure 7B ;
- les figures 9A et 9B sont des distributions du nombre de couples de souches non reliées (distribution supérieure) et du nombre de couples de souches reliées (distribution inférieure) pour l'espèce bactérienne *Staphylococcus aureus*, la figure 9B étant un agrandissement entre 0 et 0,1 de la figure 9A ;
- les figures 10A et 10B illustrent, pour l'espèce *Staphylococcus aureus,* les distances génomiques pour différentes valeurs optimales d'indice de qualité, dont la sensibilité, la spécificité, la précision, la justesse, le F1 score, l'indice de Youden, et le coefficient de corrélation de Matthews, la figure 10B étant un agrandissement entre 0 et 0,1 de la figure 10B ;

## DESCRIPTION DETAILLEE DE L'INVENTION

Dans ce qui suit « inférieur » signifie « inférieur ou égal » et même « supérieur» signifie « supérieur ou égal » sauf s'il est stipulé strictement.

Il va à présent être décrit un mode de réalisation de l'invention en relation avec la détection et le suivi de foyers infectieux microbiologiques d'une espèce bactérienne particulière dans un hôpital.

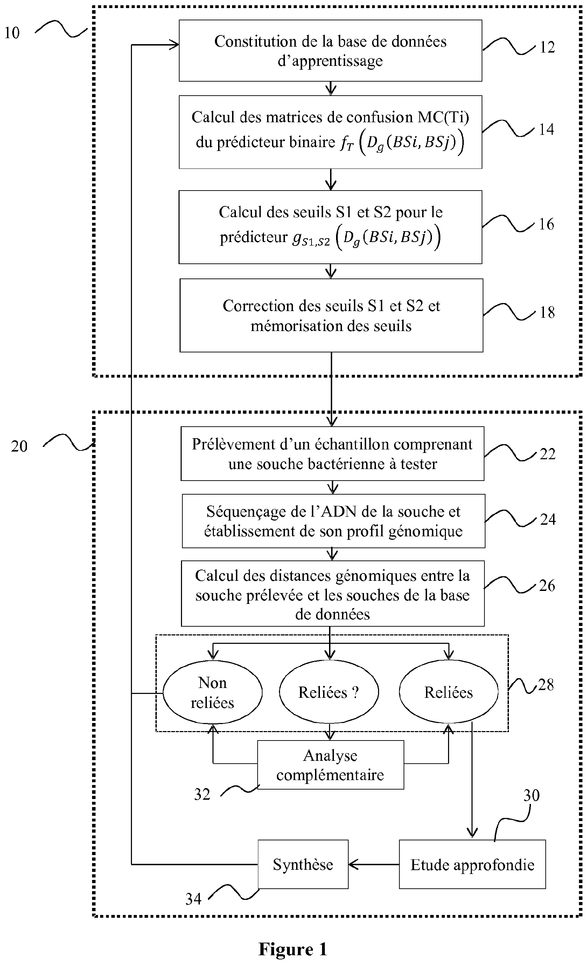

En se référant à la figure 1, ce procédé comporte une première étape **10** d'apprentissage d'au moins deux seuils, notés *S1* et *S2,* sur la base desquels des comparaisons de génomes sont réalisées pour déterminer si une souche bactérienne appartient à non à un foyer bactérien, et une seconde étape **20** de mise en oeuvre du procédé selon l'invention, paramétré avec les seuils appris lors de l'étape **10**. Plus particulièrement, le procédé se fonde sur la comparaison d'une distance génomique, notée $D_g(BSi, BSj)$ entre deux souches, notées *BSi* et *BSj,*

L'étape **10** débute par la constitution, en **12,** d'une base de données d'apprentissage pour l'espèce considérée comprenant :

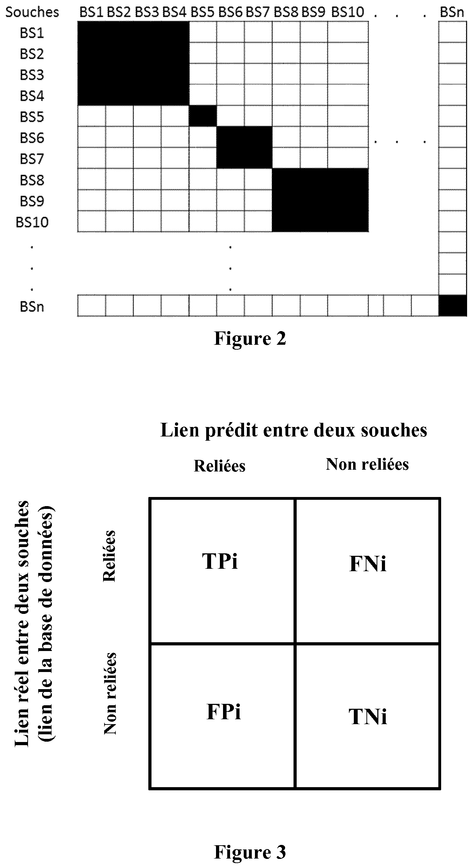

- des génomes numériques de différentes souches BS1, BS2, BS3...., BSN appartenant à l'espèce ;
- une table de correspondance, illustrée à la figure 2, reliant chaque souche de la base de données à l'ensemble des autres souches, chaque lien entre deux souches de la base pouvant prendre un état « reliées » (cases noires) lorsque les deux souches ont été préalablement déterminée comme appartenant à un même foyer bactérien, et un état « non reliées » (cases blanches) lorsque les deux souches ont été préalablement déterminées comme n'appartenant pas à un même foyer bactérien, l'état du lien entre deux souches étant par exemple déterminé lors d'une étude épidémiologique antérieure. En outre, le lien d'une souche par rapport à elle-même est fixée à l'état « reliées ». Comme visible à la figure 2, plusieurs foyers infectieux pour l'espèce considérée peuvent être pris en compte pour déterminer les états « reliées » et « non reliées » des souches de la base de données d'apprentissage. Comme il sera décrit par la suite, la base de données d'apprentissage peut également contenir des souches déterminées comme étant « reliées » sans pour autant avoir été diagnostiquée comme appartenant à un quelconque foyer bactérien. De préférence, ladite table mémorise également les distances génomiques $D_g(BSi, BSj)$ entre chaque paire de souches *BSi* et *BSj* de la base de données d'apprentissage ;
- une table qui répertorie l'ensemble des foyers infectieux identifiés avec leurs souches associées ;
- les résistomes (ensemble de marqueurs génétiques contribuant à la sensibilité ou la résistance aux antibiotiques d'une bactérie) et les virulomes (ensemble de marqueurs génétiques contribuant à la virulence d'une bactérie) des souches BS1, BS2, BS3...., BSN ;

Le génome d'une souche bactérienne est de préférence obtenu par :

- le prélèvement d'un échantillon chez un patient comprenant la souche;
- la préparation d'un isolat de la souche, par exemple en étalant l'échantillon sur un milieu de culture gélosé et en réalisant une incubation de manière à faire pousser une colonie de la souche bactérienne ;
- le prélèvement d'une partie de la colonie et la préparation de la quantité prélevée pour un séquençage (e.g. une lyse pour libérer l'ADN des bactéries, le cas échéant amplification de l'ADN libéré et la préparation d'une librairie pour les techniques de séquençage le nécessitant)
- le séquençage, de préférence complet (ou séquençage WGS), de l'ADN de manière à produire des séquences numériques, communément appelées « read », par exemple à l'aide d'une technologie de type « next génération sequencing » telle qu'avec la plateforme de séquençage « MiSeq » de la société Illumina Inc., San Diego, Californie;
- optionnellement, l'assemblage des reads de manière à produire des séquences assemblées, connues sous le terme de « contig » ;
- la caractérisation selon la technique wgMLST (pour « whole genome multilocus sequensing typing ») du génome sous forme de contig ou de reads, communément appelé « profil wgMLST ». Comme cela est connu en soi, cette caractérisation consiste à localiser des loci dans le génome parmi un ensemble prédéterminé de loci, et pour chaque locus identifié, à déterminer l'allèle qui représente ce locus. La technique wgMLST est par exemple décrite dans le document « MLST revisited: the gene-by-gene approach to bacterial genomics» de Martin C.J. Maiden, Nature Reviews Microbiology, 2013.

L'apprentissage se poursuit par le calcul de seuils *S1* et *S2* en fonction de la base de données d'apprentissage. Plus particulièrement, ce calcul consiste à transformer :

- un premier prédicteur $f_T$ d'appartenance ou non de deux souches à un foyer bactérien sur la base d'un unique seuil *T* sur les distances génomiques $D_g(BSi, BSj)$ divisant l'espace des distances génomiques en deux intervalles uniquement:

$$f_T\left(D_g(BSi, BSj)\right) = \begin{cases} 1 \text{ si les souches } BSi \text{ et } BSj \text{ sont reliées} \\ -1 \text{ si les souches } BSi \text{ et } BSj \text{ sont non reliées} \end{cases}$$

- en un second prédicteur $g_{S1,S2}$ d'appartenance ou non de deux souches à un foyer bactérien sur la base de deux seuils *S1* et *S2* sur les distances génomiques $D_g(BSi, BSj)$ divisant l'espace des distances génomiques en trois intervalles :

$$g_{S1,S2}\left(D_g(BSi, BSj)\right) = \begin{cases} 1 \text{ si les souches } BSi \text{ et } BSj \text{ sont reliées} \\ 0 \text{ si les touches } BSi \text{ et } BSj \text{ sont potentiellement reliées} \\ -1 \text{ si les souches } BSi \text{ et } BSj \text{ sont non reliées} \end{cases}$$

Dans une variante privilégiée, le premier prédicteur $f_T$ est défini tel que :

$$\begin{cases} \text{si } D_g(BSi, BSj) \leq T \text{ alors } f_T = 1 \\ \text{si } D_g(BSi, BSj) > T \text{ alors } f_T = -1 \end{cases}$$

et le second prédicteur est défini tel que:

$$\begin{cases} \text{si } D_g(BSi, BSj) \leq S1 \text{ alors } g_{S1,S2} = 1 \\ \text{si } S1 < D_g(BSi, BSj) \leq S2 \text{ alors } g_{S1,S2} = 0 \\ \text{si } D_g(BSi, BSj) > S2 \text{ alors } g_{S1,S2} = -1 \end{cases}$$

De préférence, la distance génomique $D_g(BSi, BSj)$ est une distance normalisée, et donc comprise entre 0 et 1, calculée en :

    a. identifiant dans les profils wgMLST des deux souches *BSi* et *BSj* les loci qu'elles ont en commun ;
    b. pour chaque locus commun, en déterminant s'il existe une différence allélique entre les deux souches, et dans ce cas en incrémentant de 1 un compteur *Compt* de différences alléliques si au moins une différence allélique est constatée;
    c. en calculant $D_g(BSi, BSj)$ selon la formule, avec $N_{lc}$ est le nombre de loci en commun:

$$D_g(BSi, BSj) = \frac{Compt}{N_{lc}}$$

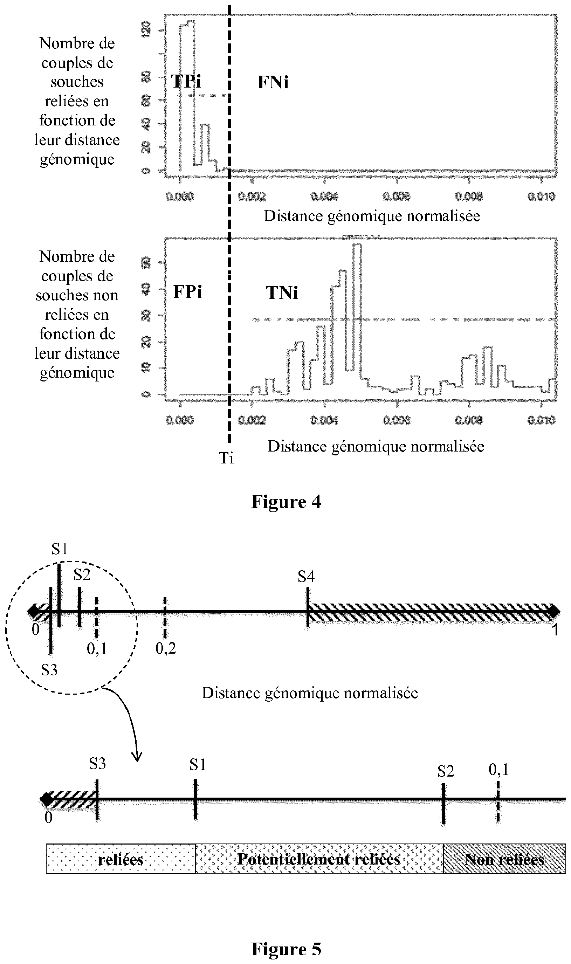

Le calcul des seuils *S1* et *S2* débute, en **14,** par le calcul d'une matrice de confusion *MC(Ti)* du prédicteur binaire $f_T$ pour chacune des valeurs *Ti* d'un ensemble *{T1,T2, ... ,TM}* de valeurs de seuils T comprises entre 0 et 1, par exemple avec un incrément de $10^{-4}$. Le calcul de la matrice de confusion *MC(Ti)*, illustrée à la figure 3, pour le seuil *Ti* est illustrée à la figure 4 et consiste à compter :

- les vrais positifs, notés « TPis », égal au nombre total de couples de souches reliées de la base telles que $D_g(BSi, BSj) \leq Ti$ ;
- les faux négatifs, notés « FNi », égal au nombre total de couples de souches reliées de la base telles que $D_g(BSi, BSj) > Ti$ ;
- la faux positifs, noté « FPi », égal au nombre total de couples de souches non reliées de la base telles que $D_g(BSi, BSj) \leq Ti$ ; et
- les vrais négatifs, notés « TNi », égal au nombre total de couples de souches non reliées de la base telles que $D_g(BSi, BSj) \leq Ti$ .

Une fois l'ensemble des matrices de confusion $\{MC(T1), MC(T2), ... , MC(TM))\}$ calculé, le procédé se poursuit, en **16,** par le calcul de différent seuils illustrés à la figure 5:

- un seuil S3 tel que la spécificité du prédicteur $f_T$ est maximale, et donc tel que la spécificité de la prédiction que deux souches sont reliées est maximale, c'est-à-dire $$S3 = arg \max_{Ti} \left( \frac{TNi}{N} \right)$$ , où N est le nombre de couples de souches non reliées ;

- un seuil S4 tel que la sensibilité du prédicteur $f_T$ est maximale, et donc tel que la sensibilité de la prédiction que deux souches sont non reliées est maximale, c'est-à-dire $$S4 = arg \max_{Ti} \left( \frac{\bar{TPi}}{P} \right)$$ , où P est le nombre de couples de souches reliées;

- le seuil S1 optimisant un premier index de qualité du prédicteur $f_T$, différent de la sensibilité et de la spécificité et tenant compte explicitement du déséquilibre entre les nombres P et N, préférentiellement le coefficient de corrélation de Matthews (« MCC »), c'est-à-dire $$S2 = arg \max_{Ti} \left( \frac{TPi.TNi - FPi.FNi}{\sqrt{(TPi+FPi).(TPi+FNi).(TNi+FPi).(TNi+FNi)}} \right)$$ ;

- le seuil S2 optimisant un second index de qualité du prédicteur $f_T$, différent de la sensibilité et de la spécificité, préférentiellement l'index de Youden, c'est-à-dire $$S1 = arg \max_{Ti} \left( \frac{TPi}{P} + \frac{\bar{TNi}}{N} - 1 \right)$$ .

Une étape *18* de contrôle de la qualité des seuils S1 et S2 est ensuite mise en oeuvre. Plus particulièrement :

- si les seuils S1 et S2 sont inférieurs ou égaux à 0,1, ils sont conservés, signifiant que la base de données d'apprentissage est appropriée pour leur calcul et leur usage ultérieur ;
- si les seuils S1 et S2 sont supérieurs à 0,1 ou diffèrent de moins de 1% , alors leurs valeurs sont fixées à 0,1 et M = max($D_g(BSi, BSj)\backslash D_g(BSi, BSj)$ < 0,2), où max($D_g(BSi, BSj)\backslash D_g(BSi, BSj)$ < 0,2) est ici la distance génomique maximale qui est la plus proche de 0,2 parmi les couples de souches reliées de la base de données d'apprentissage ;
- si l'un des seuils S1 ou S2 est supérieur à 0,1, ce seuil est alors fixé à au minimum des valeur 0,1 et max($D_g(BSi, BSj)\backslash D_g(BSi, BSj)$ < 0,2) si cette valeur minimale est différente de l'autre seuil (e.g. diffère de plus de 1%), sinon ce seuil est fixé au maximum de ces deux valeurs.

Par soucis de simplification, il sera supposé par la suite que le seuil S1 est inférieur au seuil S2, de sorte que, comme illustré à la figure 4, ces seuils divisent l'espace des distances génomiques en trois intervalles :

- un intervalle inférieur ]0, S1]. Si la distance génomique entre deux souches est comprise dans cet intervalle, ces souches sont prédites comme étant « reliées » ($g_{S1,S2}$ = 1);
- un intervalle supérieur ]S2, 1]. Si la distance génomique entre deux souches est comprise dans cette intervalle, ces souches sont prédites comme étant « non reliées » ($g_{S1,S2}$ = -1) ; et
- un intervalle intermédiaire ]S1, S2]. Si la distance génomique entre deux souches est comprise dans cette intervalle, ces souches sont prédites comme « potentiellement reliées » ( $g_{S1,S2}$ = 0) .

Les seuils S1 et S2 sont alors mémorisés dans une mémoire informatique d'un système informatique utilisé pour mettre en oeuvre l'étape **20** à présent décrite, ledit système comprenant en outre la base de données d'apprentissage. L'étape **20,** qui prend place au sein de l'hôpital pour détecter et surveiller les épidémies de nature bactérienne, est par exemple mise en oeuvre de manière systématique dès qu'un patient est atteint d'une infection bactérienne qu'un échantillon environnemental comprend une bactérie pathogène ou qu'un patient présente des symptômes identiques ou similaires à un autre patient au sein de l'hôpital. D'autres critères peuvent bien entendu être employés pour lancer cette étape.

L'étape **20** débute, en **22,** par le prélèvement d'un échantillon contenant la souche pathogène, si ce prélèvement n'a pas encore eu lieu, puis se poursuit, en **24,** par le séquençage de la souche et l'établissement de son profil wgMLST tel que décrit en relation avec l'étape **12**. En **26,** la distance génomique $D_g(BSi, BSj)$ entre la souche prélevée et chacune des souches de la base de données d'apprentissage est ensuite calculée. Un premier diagnostic épidémiologique est alors émis en **28**. Plus particulièrement :

- si la souche prélevée n'est reliée à aucune souche de la base de données, c'est-à-dire que quel que soit la souche de la base de données la distance génomique $D_g(BSi, BSj)$ avec la souche prélevée est supérieure au seuil S2, alors il est déterminé que la souche prélevée n'appartient à aucun foyer bactérien ;
- si la souche prélevée est reliée à une souche de la base de données, c'est-à-dire que ces deux souches ont une distance génomique $D_g(BSi, BSj)$ inférieure ou égale au seuil S1, une alarme est déclenchée à l'attention de l'utilisateur et une étude épidémiologique approfondie **30** est lancée ainsi que, le cas échéant, des mesures prophylactique pour lutter contre la transmission de la souche prélevée au sein de l'hôpital ;
- si la souche prélevée est potentiellement reliée à une souche de la base de données (noté « reliées ? » dans la figure 1), c'est-à-dire que ces deux souches ont une distance génomique $D_g(BSi, BSj)$ comprise entre les seuils S1 et S2, une analyse complémentaire est menée, en **34,** pour lever l'incertitude sur le lien entre ces deux souches. De manière préférentielle, le résistome et le virulome de la souche prélevée est déterminé puis comparé au résistome et le virulome de la souche à laquelle elle est potentiellement reliée. Si les résistomes et les virulomes sont concordants, les souches sont alors déterminées comme étant reliées, l'alarme est déclenchée et l'étude approfondie **30** menée. Dans le cas contraire, les souches sont déterminées comme étant non reliées. Enfin dans le cas où cette comparaison ne permet pas de trancher, l'étude approfondie **30** est menée. D'autres informations peuvent être utilisées lors de cette étude complémentaire, comme par exemple la durée écoulée entre le prélèvement et celui de la souche de la base de données, le nombre de SNP différents dans les gènes plastiques, etc.

Comme cela est connu en soi, un des objectifs de l'étude **30,** menée par l'équipe d'épidémiologie de l'hôpital, est de déterminer si différentes souches prélevées au sein de l'hôpital constituent une épidémie. A l'issue de cette étude, le lien entre différentes souches est définitivement établi, à savoir « reliées » ou « non reliées ». Si par ailleurs une épidémie est détectée alors les souches de l'épidémie sont également étiquetées en fonction de cette épidémie. Le génome, le profils wgMLST, le resistome et le virulome de la souche prélevée, ses liens avec les autres souches de la base de données ainsi que les informations concernant le foyer bactérien sont ensuite mémorisés dans la base de données d'apprentissage pour pouvoir être utilisés ultérieurement. Les seuils S1 et S2 peuvent être ainsi actualisés régulièrement ou à chaque nouvelle entrée dans la base de données afin d'affiner leurs valeurs.

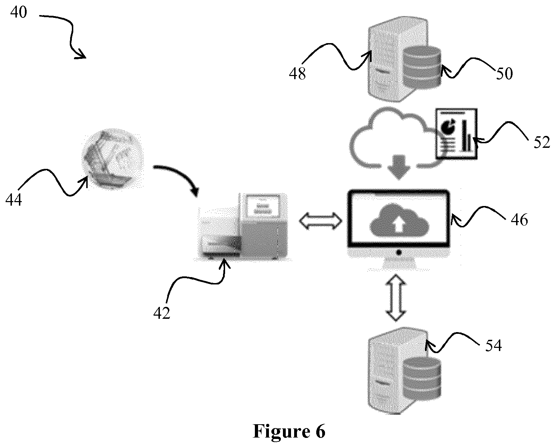

La figure 6 illustre un système informatique et de séquençage **40** pour la mise en oeuvre du procédé selon l'invention. Le système **40** comporte une plateforme de séquençage **42** pour séquencer l'ADN bactérien d'un échantillon **44** et ainsi produire un ensemble de séquence numériques, ou « reads ». La plateforme **42** est connectée à une unité de traitement d'information **46,** par exemple un ordinateur personnel, qui reçoit les séquences, et met optionnellement un programme d'assemblage des reads pour produire des contig. L'unité **46** est par ailleurs connectée à un serveur distant **48** mettant en oeuvre un logiciel en tant que service (ou « Saas »), par exemple sous la forme d'une solution cloud. L'unité **46,** sur lequel tourne un logiciel « front end », envoie au serveur **48** les génomes séquencés par la plateforme **42** sous forme de reads ou de contigs. Le serveur **48,** sur lequel tourne le service informatique sous forme de backend et qui est connecté à la base de données d'apprentissage **50,** reçoit les génomes et met en oeuvre les étapes de traitement du procédé selon l'invention (e.g. les étapes 14-18 et 24-32 de la figure 1), le serveur mémorisant dans une mémoire informatique l'ensemble des instructions nécessaires à cette mise en oeuvre. Le serveur renvoie les résultats du traitement à l'unité **46** sous la forme d'un rapport **52**. Le système **40** comporte également un ou plusieurs serveurs **54** connecté(s) à l'unité **42,** ces serveurs étant notamment ceux du système informatique mémorisant les données patients et épidémiologiques, ces données étant utilisées lors des études approfondies pour caractériser les foyers bactériens épidémiologiques.

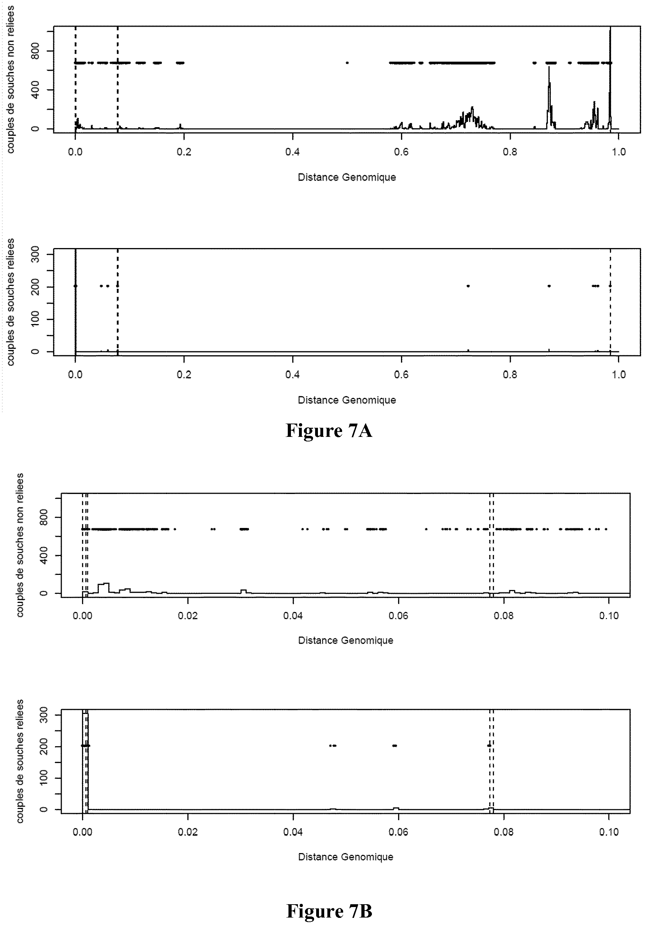

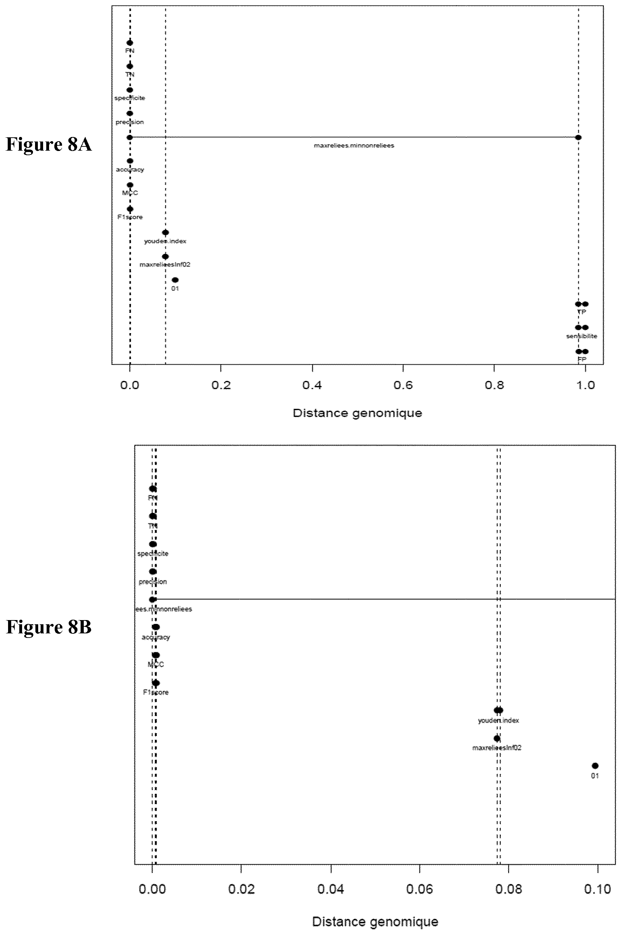

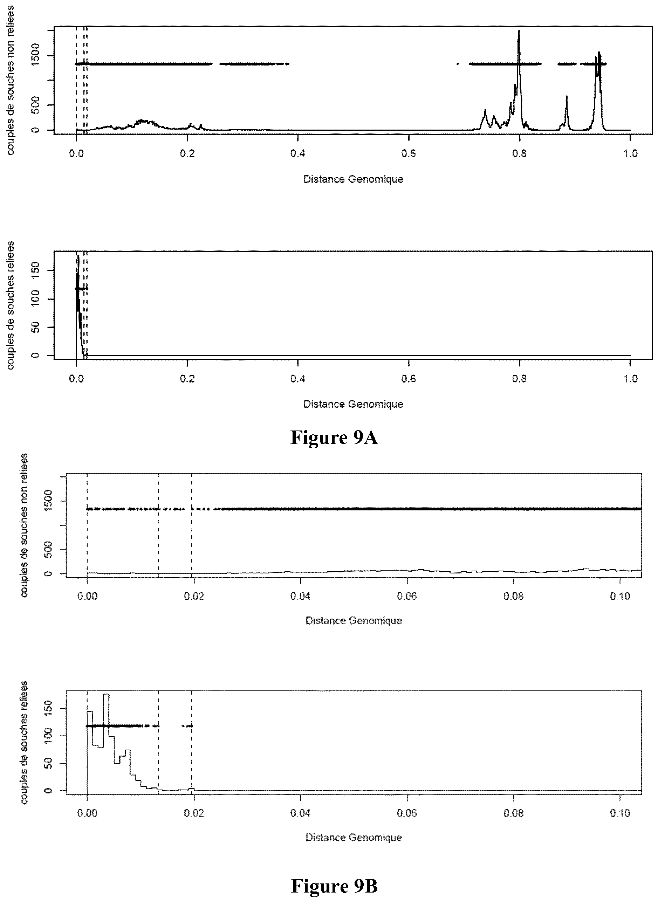

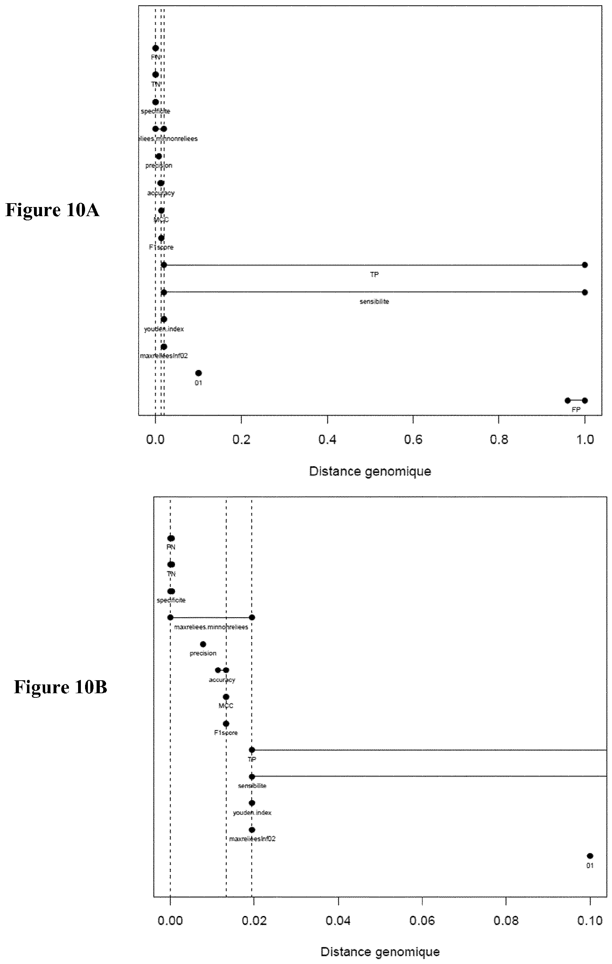

Les figures 7 et 9 illustrent des distributions du nombre de couples de souches reliées et de souches non reliées respectivement pour l'espèce *Clostridium difficile* (figures 7A et 7B) et *Staphylococcus aureus* (figures 9A et 9B). Comme on peut le noter sur ces figures, il existe des couples de souches reliées dont la distance génomique est importante (par exemple au-delà de 0,6 pour *Clostridium difficile*) et des couples de souches non reliées dont la distance génomique est faible (par exemple en deçà de 0,2 pour *Staphylococcus aureus*). Il existe ainsi une zone dans laquelle une distance génomique pourrait coder à la fois pour l'état « reliées » ou l'état « non reliées » si un seul seuil était employé. Cette zone intermédiaire est naturellement présente et correspond par exemple à des souches appartenant à un même sous-type mais n'ayant pas été jugées comme appartenant à un même foyer bactérien. De même, on observe au travers des figures 8A-B et 10A-B, qu'en choisissant les seuils S3 (spécificité maximale, noté « sensibilité ») et S4 (sensibilité maximale, notée « spécificité ») pour diviser en trois l'espace des distances génomiques, la zone intermédiaire est si importante que bon nombre de souches seraient jugées comme potentiellement reliées. En utilisant les seuils S1 (e.g. maximisant le coefficient de Matthews MMC) et S2 (e.g. optimisant l'indice de Youden) qui optimisent la qualité de la prédiction, on note que la zone intermédiaire est sensiblement réduite tout en conservant une très bonne sensibilité globale.

Il a été décrit une application à l'épidémiologique de bactérie pathogène au sein d'un hôpital. Evidemment, l'invention n'est pas limitée à cette application et peut être employé dans le domaine du contrôle microbiologique industriel (par exemple le contrôle agro-alimentaire), environnemental et vétérinaire.

Il a été décrit l'emploi de profil wgMLST pour calculer les distances génomiques. D'autres profils peuvent être employés, comme par exemple des profils cgMLST (« core genome multilocus sequencing typing »), MLST, des ensembles de SNP ou de gènes.

Il a été décrit l'emploi de l'indice de Youden et du coefficient de corrélation de Matthews. D'autres indices de qualités peuvent être employés, comme par exemple le F1 score (i.e. 2 $TP/(2TP + FP + FN)$ ), le coefficient $\chi_1$ , la justesse (ou « accuracy », i.e. $(TP + TN)/(N + P)$), la précision (i.e. $TP/(TP + FP)$). De préférence au moins 1 de ces indices tient compte du déséquilibre de la base de données.

Il a été décrit une base de données d'apprentissage utilisée également pour la comparaison avec des souches prélevées. En variante, une base de données séparée, ou « base de données épidémiologique », peut être employée pour traiter les souches prélevées. Une telle base est par exemple propre à un hôpital, une institution, une société ou autre, et la base de donnée d'apprentissage est alors utilisée uniquement pour établir la valeur des seuils.

## Revendications

1. Procédé de détection et de surveillance d'un foyer bactérien lié à une espèce bactérienne au sein d'une zone géographique, le procédé étant mis en oeuvre par ordinateur et comprenant :

   - l'obtention d'un génome numérique d'une souche bactérienne prélevée au sein de la zone géographique et appartenant à l'espèce bactérienne ;
   - le calcul d'une distance génomique du génome numérique obtenu avec un génome numérique d'une base de données, dite « épidémiologique », comprenant au moins un génome numérique d'une souche bactérienne appartenant à l'espèce bactérienne;
   - la prédiction :

     ◦ que la souche bactérienne prélevée et la souche bactérienne de la base de données appartiennent au foyer bactérien si leur distance génomique est inférieure à un premier seuil prédéterminé ; ou
     ◦ que la souche bactérienne prélevée et la souche bactérienne de la base de données n'appartiennent pas au foyer bactérien si leur distance génomique est supérieure à un second seuil prédéterminé strictement supérieur au premier seuil ; ou
     ◦ que la souche bactérienne prélevée et la souche bactérienne de la base de données appartiennent peut être au foyer bactérien si leur distance génomique est comprise entre le premier et le second seuil ;

   procédé selon lequel :

   - le premier seuil est supérieur ou égal à un troisième seuil tel qu'une prédiction d'appartenance au foyer bactérien de deux souches bactériennes ayant une distance génomique inférieure au troisième seuil a une spécificité maximale ; et
   - le second seuil est inférieur ou égal à un quatrième seuil tel qu'une prédiction de non appartenance au foyer bactérien de deux souches bactériennes ayant une distance génomique supérieure au quatrième seuil a une sensibilité maximale.

2. Procédé selon la revendication 1, dans lequel le premier et le second seuils sont égaux à deux distances génomiques calculées :

   - en constituant une base de données d'apprentissage de génomes numériques de souches bactériennes appartenant à l'espèce bactérienne, ladite base comprenant :

     ◦ des couples de souches bactériennes préalablement déterminées comme appartenant à un même foyer bactérien, et étiquetés comme « couples de souches reliées » ;

○ des couples de souches bactériennes préalablement déterminée comme n'appartenant pas à un même foyer bactérien, et étiquetés comme « couples de souches non reliées » ;

- en choisissant un prédicteur binaire configuré pour prédire que deux souches bactériennes sont reliées ou non reliées par comparaison de leur distance génomique à un cinquième seuil ;
- pour chaque valeur de cinquième seuil appartenant à un ensemble prédéterminé de valeurs de cinquième seuil, en calculant

○ une matrice de confusion dudit prédicteur en fonction de la base de données d'apprentissage ;
○ un premier index de qualité du prédicteur en fonction de la matrice de confusion, ledit premier index étant différent de la sensibilité et de la spécificité du prédicteur ;
○ un second index de qualité, différent du premier index, en fonction de la matrice de confusion, ledit second index étant différent du premier index, de la sensibilité et de la spécificité du prédicteur ;

- en recherchant une première valeur de cinquième seuil qui optimise le premier index et une seconde valeur de cinquième seuil qui optimise le second index ;
- en posant le premier seuil comme égal au minimum de la première et de la seconde valeurs de cinquième seuil et en posant le second seuil comme égal au maximum de la première et de la seconde valeurs de cinquième seuil.

3. Procédé selon la revendication 2, dans lequel le premier index est choisi pour tenir compte du déséquilibre, dans la base de données d'apprentissage, entre le nombre de couples de souches reliées et le nombre de couples de souches reliées.

4. Procédé selon la revendication 3, dans lequel le premier index est le coefficient de corrélation de Matthews ou le F1 score.

5. Procédé selon l'une des revendications 2 à 4, dans lequel le second index est l'index de Youden.

6. Procédé selon l'une des revendications 2 à 5, dans lequel le prédicteur est choisi de sorte que :

- les vrais positifs correspondent aux couples de souches reliées ayant une distance génomique inférieure au cinquième seuil :
- les faux négatifs correspondent aux couples de souches reliées ayant une distance génomique supérieure au cinquième seuil ;
- la faux positifs correspondent aux couples de souches non reliées ayant une distance génomique inférieure au cinquième seuil ; et
- les vrais négatifs correspondent aux couples de souches non reliées ayant une distance génomique supérieure au cinquième seuil.

7. Procédé selon l'une des revendications 2 à 6, dans lequel la base de données épidémiologique comprend la base de données d'apprentissage.

8. Procédé selon l'une quelconque des revendications précédentes, dans lequel la distance génomique est une distance normalisée.

9. Procédé selon la revendication 8, dans lequel la distance génomique entre deux souches bactériennes est calculée en :

- sélectionnant, dans un ensemble prédominé de loci, les loci communs aux génomes numériques desdites souches ;
- comptant le nombre de différences alléliques, aux loci communs, entre les deux génomes numériques desdites souches ;
- en divisant ledit nombre de différences par le nombre de loci communs.

10. Procédé selon la revendication 9 et l'une des revendications 4 ou 5, dans lequel si les premières et secondes valeurs de cinquième seuil sont supérieures à 0,1, alors :

- le second seuil est posé égal à 0,1 ;
- le premier seuil est posé égal à max($D_g \backslash D_g < 0,2$), où max($D_g \backslash D_g < 0,2$) est la plus grande distance génomique, parmi les couples de souches reliées, strictement inférieure à 0,2.

11. Procédé selon l'une quelconque des revendications précédentes, dans lequel les distances entre les génomes numériques sont calculées en fonction d'une base wgMLST, cgMLST, MLST, de gènes ou de SNP.

12. Procédé selon l'une quelconque des revendications précédentes, dans lequel lorsqu'une souche prélevée est prédite comme appartenant au foyer bactérien, elle est étiquetée dans la base de donnée épidémiologique comme étant « reliée » avec les souches bactériennes du foyer bactérien et comme étant « non reliée » avec les autres souches bactériennes.

13. Procédé selon l'une quelconque des revendications précédentes, dans lequel lorsqu'une souche prélevée est prédite comme appartenant peut-être au foyer bactérien, une caractérisation supplémentaire de ladite souche est mise en oeuvre pour déterminer si elle appartient effectivement audit foyer, et si tel est le cas la souche bactérienne prélevée est étiquetée, dans la base de donnée épidémiologique, comme étant « reliée » avec les souches bactériennes du foyer bactérien et comme étant « non reliée » avec les autres souches bactériennes.

14. Procédé selon l'une quelconque des revendications précédentes, dans lequel le premier et le second seuil sont recalculés régulièrement et/ou dès que $N$ nouvelles souches sont ajoutées à la base de données épidémiologique, où $N$ est un entier supérieur ou égal à 1.

15. Procédé selon l'une quelconque des revendications précédentes, dans lequel lorsqu'une souche est prédite comme appartenant au foyer bactérien, des mesures prophylactiques sont mises en oeuvre pour enrailler ledit foyer.

**Patentansprüche**

1. Verfahren zur Erkennung und Überwachung eines mit einer Bakterienspezies zusammenhängenden Bakterienherds innerhalb einer geographischen Zone, wobei das Verfahren computerimplementiert wird und enthält:

   - den Erhalt eines digitalen Genoms eines in der geographischen Zone entnommenen und zur Bakterienspezies gehörenden Bakterienstamms;
   - die Berechnung einer genomischen Distanz des erhaltenen digitalen Genoms zu einem digitalen Genom einer "epidemiologisch" genannten Datenbank, die mindestens ein digitales Genom eines zur Bakterienspezies gehörenden Bakterienstamms enthält;
   - die Vorhersage:

      ◦ dass der entnommene Bakterienstamm und der Bakterienstamm der Datenbank zum Bakterienherd gehören, wenn ihre genomische Distanz niedriger ist als eine erste vorbestimmte Schwelle; oder
      ◦ dass der entnommene Bakterienstamm und der Bakterienstamm der Datenbank nicht zum Bakterienherd gehören, wenn ihre genomische Distanz höher ist als eine zweite vorbestimmte Schwelle strikt höher als die erste Schwelle; oder
      ◦ dass der entnommene Bakterienstamm und der Bakterienstamm der Datenbank vielleicht zum Bakterienherd gehören, wenn ihre genomische Distanz zwischen der ersten und der zweiten Schwelle liegt;

   Verfahren, gemäß dem:

   - die erste Schwelle höher als eine oder gleich einer dritten Schwelle ist, derart, dass eine Zugehörigkeitsvorhersage zum Bakterienherd von zwei Bakterienstämmen, die eine genomische Distanz niedriger als die dritte Schwelle haben, eine maximale Spezifität hat; und
   - die zweite Schwelle niedriger als eine oder gleich einer vierten Schwelle ist, derart, dass eine Nichtzugehörigkeitsvorhersage zum Bakterienherd von zwei Bakterienstämmen, die eine genomische Distanz niedriger als die vierte Schwelle haben, eine maximale Sensitivität hat.

2. Verfahren nach Anspruch 1, wobei die erste und die zweite Schwelle gleich zwei genomischen Distanzen sind, die berechnet werden:

- durch Erstellen einer Lerndatenbank von digitalen Genomen von Bakterienstämmen, die zu der Bakterienspezies gehören, wobei die Bank enthält:

  ∘ Paare von Bakterienstämmen, die vorab als zu einem gleichen Bakterienherd gehörend bestimmt werden und als "verbundene Paare von Stämmen" eingeordnet werden;
  ∘ Paare von Bakterienstämmen, die vorab als nicht zu einem gleichen Bakterienherd gehörend bestimmt werden und als "nicht verbundene Paare von Stämmen" eingeordnet werden;

- durch Auswahl eines binären Prädiktors, der konfiguriert ist, durch Vergleich ihrer genomischen Distanz mit einer fünften Schwelle vorherzusagen, dass zwei Bakterienstämme verbunden oder nicht verbunden sind;
- für jeden Wert fünfter Schwelle, der zu einer vorherbestimmten Einheit von Werten fünfter Schwelle gehört, durch Berechnen

  ∘ einer Konfusionsmatrix des Prädiktors abhängig von der Lerndatenbank;
  ∘ eines ersten Qualitätsindex des Prädiktors abhängig von der Konfusionsmatrix, wobei der erste Index anders ist als die Sensitivität und die Spezifität des Prädiktors;
  ∘ eines zweiten Qualitätsindex, anders als der erste Index, abhängig von der Konfusionsmatrix, wobei der zweite Index anders als der erste Index, die Sensitivität und die Spezifität des Prädiktors ist;

- durch Suche nach einem ersten Wert fünfter Schwelle, der den ersten Index optimiert, und einem zweiten Wert fünfter Schwelle, der den zweiten Index optimiert;
- durch Festlegen der ersten Schwelle als gleich dem Minimum des ersten und des zweiten Werts fünfter Schwelle und durch Festlegen der zweiten Schwelle als gleich dem Maximum des ersten und des zweiten Werts fünfter Schwelle.

3. Verfahren nach Anspruch 2, wobei der erste Index ausgewählt wird, um das Ungleichgewicht, in der Lerndatenbank, zwischen der Anzahl von Paaren von verbundenen Stämmen und der Anzahl von Paaren von verbundenen Stämmen zu berücksichtigen.

4. Verfahren nach Anspruch 3, wobei der erste Index der Matthews-Korrelationskoeffizient oder der F1-Score ist.

5. Verfahren nach einem der Ansprüche 2 bis 4, wobei der zweite Index der Youden-Index ist.

6. Verfahren nach einem der Ansprüche 2 bis 5, wobei der Prädiktor derart gewählt wird, dass:

   - die True Positives den Paaren von verbundenen Stämmen entsprechen, die eine genomische Distanz niedriger als die fünfte Schwelle haben:
   - die False Negatives den Paaren von verbundenen Stämmen entsprechen, die eine genomische Distanz höher als die fünfte Schwelle haben;
   - die False Positives den Paaren von nicht verbundenen Stämmen entsprechen, die eine genomische Distanz niedriger als die fünfte Schwelle haben; und

   die True Negatives den Paaren von nicht verbundenen Stämmen entsprechen, die eine genomische Distanz höher als die fünfte Schwelle haben.

7. Verfahren nach einem der Ansprüche 2 bis 6, wobei die epidemiologische Datenbank die Lerndatenbank enthält.

8. Verfahren nach einem der vorhergehenden Ansprüche, wobei die genomische Distanz eine normierte Distanz ist.

9. Verfahren nach Anspruch 8, wobei die genomische Distanz zwischen zwei Bakterienstämmen berechnet wird durch:

   - Auswahl, in einer prädominierten Einheit von Loci, der den digitalen Genomen der Stämme gemeinsamen Loci;
   - Zählen der Anzahl von allelischen Differenzen, an den gemeinsamen Loci, zwischen den zwei digitalen Genomen der Stämme;
   - Teilen der Anzahl von Differenzen durch die Anzahl von gemeinsamen Loci.

10. Verfahren nach Anspruch 9 und einem der Ansprüche 4 oder 5, wobei, wenn die ersten und zweiten Werte fünfter Schwelle höher sind als 0,1, gilt:

- die zweite Schwelle wird gleich 0,1 festgelegt;
- die erste Schwelle wird gleich max($D_g$\\$D_g$ < 0,2) festgelegt, wobei max($D_g$\\$D_g$ < 0,2) die größte genomische Distanz unter den Paaren von verbundenen Stämmen ist, strikt niedriger als 0,2.

11. Verfahren nach einem der vorhergehenden Ansprüche, wobei die Distanzen zwischen den digitalen Genomen abhängig von einer Basis wgMLST, cgMLST, MLST, von Genen oder von SNP berechnet werden.

12. Verfahren nach einem der vorhergehenden Ansprüche, wobei, wenn ein entnommener Stamm als zum Bakterienherd gehörend vorhergesagt wird, er in der epidemiologischen Datenbank als mit den Bakterienstämmen des Bakterienherds "verbunden" und als mit den anderen Bakterienstämmen "nicht verbunden" festgelegt wird.

13. Verfahren nach einem der vorhergehenden Ansprüche, wobei, wenn ein entnommener Stamm als vielleicht zum Bakterienherd gehörend vorhergesagt wird, eine zusätzliche Charakterisierung des Stamms durchgeführt wird, um zu bestimmen, ob er tatsächlich zum Herd gehört, und wenn dies der Fall ist, wird der entnommene Bakterienstamm in der epidemiologischen Datenbank als mit den Bakterienstämmen des Bakterienherds "verbunden" und als mit den anderen Bakterienstämmen "nicht verbunden" festgelegt.

14. Verfahren nach einem der vorhergehenden Ansprüche, wobei die erste und die zweite Schwelle regelmäßig und/oder sobald N neue Stämme zur epidemiologischen Datenbank hinzugefügt werden, neu berechnet werden, wobei N eine ganze Zahl größer als oder gleich 1 ist.

15. Verfahren nach einem der vorhergehenden Ansprüche, wobei, wenn ein Stamm als zum Bakterienherd gehörend vorhergesagt wird, prophylaktische Maßnahmen ergriffen werden, um den Herd unter Kontrolle zu bringen.

**Claims**

1. A method for detecting and monitoring a bacterial outbreak linked to a bacterial species within a geographic zone, the method being a computer implemented method and comprising:

   - obtaining a digital genome of a bacterial strain sampled within the geographic zone and belonging to the bacterial species;
   - calculating a genomic distance of the digital genome obtained from a digital genome of a database, called "epidemiological", comprising at least one digital genome of a bacterial strain belonging to the bacterial species;
   - predicting:

     ∘ that the bacterial strain sampled and the bacterial strain of the database belong to the bacterial outbreak if their genomic distance is below a first predetermined threshold; or
     ∘ that the bacterial strain sampled and the bacterial strain of the database do not belong to the bacterial outbreak if their genomic distance is above a second predetermined threshold strictly higher than the first threshold; or
     ∘ that the bacterial strain sampled and the bacterial strain of the database perhaps belong to the bacterial outbreak if their genomic distance is between the first and the second threshold;

   and according to said method:

   - the first threshold is greater than or equal to a third threshold such that a prediction that two bacterial strains having a genomic distance below the third threshold belong to the bacterial outbreak has a maximum specificity; and
   - the second threshold is less than or equal to a fourth threshold such that a prediction that two bacterial strains having a genomic distance above the fourth threshold do not belong to the bacterial outbreak has a maximum sensitivity.

2. The method as claimed in claim 1, in which the first and the second thresholds are equal to two genomic distances calculated:

   - by constructing a learning database of digital genomes of bacterial strains belonging to the bacterial species, said base comprising:

○ pairs of bacterial strains previously determined as belonging to one and the same bacterial outbreak, and tagged as "pairs of related strains";
○ pairs of bacterial strains previously determined as not belonging to one and the same bacterial outbreak, and tagged as "pairs of unrelated strains";

- by selecting a binary predictor configured for predicting that two bacterial strains are related or unrelated by comparing their genomic distance against a fifth threshold;
- for each value of fifth threshold belonging to a predetermined set of values of fifth threshold, by calculating

○ a confusion matrix of said predictor as a function of the learning database;
○ a first quality index of the predictor as a function of the confusion matrix, said first index being different than the sensitivity and specificity of the predictor;
○ a second quality index, different than the first index, as a function of the confusion matrix, said second index being different than the first index, of the sensitivity and specificity of the predictor;

- finding a first value of fifth threshold that optimizes the first index and a second value of fifth threshold that optimizes the second index;
- setting the first threshold equal to the minimum of the first and second values of fifth threshold and setting the second threshold equal to the maximum of the first and second values of fifth threshold.

3. The method as claimed in claim 2, in which the first index is selected for taking into account the imbalance, in the learning database, between the number of pairs of related strains and the number of pairs of related strains.

4. The method as claimed in claim 3, in which the first index is the Matthews correlation coefficient or the F1 score.

5. The method as claimed in one of claims 2 to 4, in which the second index is the Youden index.

6. The method as claimed in one of claims 2 to 5, in which the predictor is selected in such a way that:

- the true positives correspond to the pairs of related strains having a genomic distance below the fifth threshold:
- the false negatives correspond to the pairs of related strains having a genomic distance above the fifth threshold;
- the false positives correspond to the pairs of unrelated strains having a genomic distance below the fifth threshold; and
- the true negatives correspond to the pairs of unrelated strains having a genomic distance above the fifth threshold.

7. The method as claimed in one of claims 2 to 6, in which the epidemiological database comprises the learning database.

8. The method as claimed in any one of the preceding claims, in which the genomic distance is a normalized distance.

9. The method as claimed in claim 8, in which the genomic distance between two bacterial strains is calculated by:

- selecting, in a set predominantly of loci, the loci common to the digital genomes of said strains;
- counting the number of allelic differences, at the common loci, between the two digital genomes of said strains;
- dividing said number of differences by the number of common loci.

10. The method as claimed in claim 9 and one of claims 4 or 5, in which if the first and second values of fifth threshold are above 0.1, then:

- the second threshold is set equal to 0.1;
- the first threshold is set equal to $\max(D_g \backslash D_g < 0.2)$, where $\max(D_g \backslash D_g < 0.2)$ is the largest genomic distance, among the pairs of related strains, strictly below 0.2.

11. The method as claimed in any one of the preceding claims, in which the distances between the digital genomes are calculated as a function of a database of markers, in particular a database wgMLST, cgMLST, MLST, of genes or of SNPs.

**12.** The method as claimed in any one of the preceding claims, in which when a sampled strain is predicted as belonging to the bacterial outbreak, it is tagged in the epidemiological database as being "related" to the bacterial strains of the bacterial outbreak and as being "unrelated" to the other bacterial strains.

**13.** The method as claimed in any one of the preceding claims, in which when a sampled strain is predicted as perhaps belonging to the bacterial outbreak, an additional characterization of said strain is carried out to determine whether it actually belongs to said outbreak, and if that is so, the sampled bacterial strain is tagged, in the epidemiological database, as being "related" to the bacterial strains of the bacterial outbreak and as being "unrelated" to the other bacterial strains.

**14.** The method as claimed in any one of the preceding claims, in which the first and the second threshold are recalculated regularly and/or as soon as $N$ new strains are added to the epidemiological database, where $N$ is an integer greater than or equal to 1.

**15.** The method as claimed in any one of the preceding claims, in which when a strain is predicted as belonging to the bacterial outbreak, prophylactic measures are put in place to halt said outbreak.

**Figure 1**

Figure 2

**Lien prédit entre deux souches**

| | | Reliées | Non reliées |
|---|---|---|---|
| Lien réel entre deux souches (lien de la base de données) | Reliées | TPi | FNi |
| | Non reliées | FPi | TNi |

Figure 3

Nombre de couples de souches reliées en fonction de leur distance génomique

TPi

FNi

Distance génomique normalisée

Nombre de couples de souches non reliées en fonction de leur distance génomique

FPi

TNi

Distance génomique normalisée

Ti

**Figure 4**

S1

S2

S4

0

0,1

0,2

1

S3

Distance génomique normalisée

S3

S1

S2

0,1

0

reliées    Potentiellement reliées    Non reliées

**Figure 5**

40

48

50

44

42

52

46

54

**Figure 6**

Figure 7A

Figure 7B

Figure 8A

Distance genomique

Figure 8B

Distance genomique

**Figure 9A**

**Figure 9B**

Figure 10A

Figure 10B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **J. SIMSON et al.** *Beyond the SNP threshold : identifying outbreak clusters using inferred transmission,* Décembre 2018
- **WALKER TIMOTHY M et al.** Whole-genome sequencing to delineate Mycobacterium tuberculosis outbreaks: a rétrospective observational study. *The Lancet Infectious Diseases,* 15 Novembre 2012, vol. 13 (2), 137-146
- **MARTIN C.J. MAIDEN.** MLST revisited: the gene-by-gene approach to bacterial genomics. *Nature Reviews Microbiology,* 2013